# EUROPÄISCHE PATENTSCHRIFT

(11) **EP 1 112 524 B1**
(45) Veröffentlichungstag und Bekanntmachung des Hinweises auf die Patenterteilung: **28.05.2008**
(21) Anmeldenummer: 99944374.0
(22) Anmeldetag: 11.08.1999
(51) Int. Cl.: G02B 27/09, A61B 18/22, A61N 5/06

(54) **MEDIZINISCHES HANDSTÜCK FÜR LASERSTRAHLUNGSQUELLE**
MEDICAL HAND PIECE FOR A LASER RADIATION SOURCE
PIECE A MAIN A USAGE MEDICAL POUR SOURCE DE RAYONNEMENT LASER

(30) Priorität: 13.08.1998 DE 19836649
(43) Veröffentlichungstag der Anmeldung: 04.07.2001
(73) Patentinhaber: Asclepion Laser Technologies GmbH, 07747 Jena (DE)
(72) Erfinder: ELBRECHT, Jens, D-07751 Jena (DE); SCHRÖDER, Eckhard, D-90542 Eckental (DE); KÜHNERT, Jürgen, D-07749 Jena (DE); ZIMMERMANN, Gabriele, D-07747 Jena (DE)
(74) Vertreter: Geyer, Fehners & Partner
(86) Internationale Anmeldenummer: PCT/EP1999/005889
(87) Internationale Veröffentlichungsnummer: WO 2000/010049

(56) Entgegenhaltungen:
- WO-A-91/04829
- WO-A-95/18984
- WO-A-98/52481
- DE-A- 4 103 615
- DE-A- 19 623 749
- FR-A- 2 738 082
- US-A- 5 558 666
- US-A- 5 755 751

## Beschreibung

### Gebiet der Erfindung

Die Erfindung bezieht sich auf ein medizinisches Handstück, das über eine Strahlführungseinrichtung mit einer Laserstrahlungsquelle verbunden ist und mit dem ein Laserstrahl auf ein Behandlungsareal gerichtet wird.

### Stand der Technik

In der Dermatologie wird häufig Laserstrahlung zur Behandlung von Feuermalen, zur Entfernung von Tätowierungen, zur Hauterneuerung wie auch zur Haarentfernung genutzt. Dabei werden meist kurze Laserimpulse mit einer impulsdauer im Nano- und Mikrosekundenbereich in das Gewebe eingebracht. Derartige Behandlungen dienen vorwiegend der Verbesserung der Lebensqualität der Patienten und sind in der Regel der Kosmetik zuzuordnen.

Die medizinisch-technischen Geräte zur Durchführung solcher Behandlungen umfassen im wesentlichen eine Laserstrahlungsquelle und ein Handstück, das zur manuellen Ausrichtung der von der Laserstrahlungsquelle emittierten Strahlung auf das Zielgebiet dient.

Um eine leichtgewichtige Bauweise des Handstückes zu erzielen und so eine möglichst ungehinderte Manipulation zu ermöglichen, sind Laserstrahlungsquelle und Handstück als getrennte Baugruppen ausgeführt, wobei die Übertragung der Laserstrahlung von der Strahlungsquelle zum Handstück mittels einer beweglichen Strahlführungseinrichtung erfolgt. Die Strahlführungseinrichtung kann aus mehreren, durch Gelenke miteinander verbundenen starren Übertragungsgliedern bestehen oder auch als biegsame Faseroptik ausgebildet sein.

Die Handstücke, auf die sich die Erfindung bezieht, weisen am Übergang von der Strahlführungseinrichtung ein Einkoppelelement auf und sind mit einer Abstrahlfläche für die Laserstrahlung ausgestattet.

Bei bekannten Handstücken dieser Art erfolgt die Abstrahlung des Laserstrahles mit denselben Eigenschaften, mit denen dieser in das Handstück eingekoppelt wird, d.h. es bleiben insbesondere die Verteilung der Strahlungsintensität innerhalb des Strahlquerschnittes und die Geometrie des Strahlquerschnittes weitestgehend erhalten, und die Laserstrahlung wird auch so auf den Behandlungsort gerichtet.

Nun ist es für viele dermatologische Anwendungen, bei denen große Hautareale zu lasern sind, jedoch erforderlich, während der Behandlung die Abstrahlfläche mehrmals nebeneinander aufzusetzen, um die gesamte zu behandelnde Fläche abzudecken. Dabei ist es im Sinne einer gleichmäßigen Behandlung der gesamten Fläche von Bedeutung, daß einerseits die einzelnen Aufsetzflächen (Spots) einander nicht Überschneiden, andererseits aber auch keine unbehandelten Fehlstellen zwischen den Aufsetzflächen verbleiben.

Unter diesem Aspekt ist beispielsweise ein Laserstrahl mit kreisrundem Querschnitt ungünstig, da bei nebeneinandergesetzten kreisrunden Spots stets entweder Überschneidungen oder Fehlstellen auftreten, wodurch eine gleichmäßige Energieeinstrahlung in ein Behandlungsareal nicht möglich ist. Es ist also wünschenswert, zur effektiven Bearbeitung die Strahlform am abstrahlungsseitigen Ende des Handstückes so zu gestalten, daß bei Abrasterung eines Behandlungsareals mit mehreren Spots ein gleichmäßiger Energieeintrag gewährleistet ist.

Das gilt auch in bezug auf die Energieverteilung innerhalb des Laserstrahlquerschnittes. Ist die Energiedichte am Rand des Laserstrahlquerschnittes geringer als in dessen Zentrum, wie das beispielsweise bei einer gauß'schen Verteilung der Fall ist, ist eine gleichmäßige Einwirkung über die gesamte Fläche des Strahlquerschnittes hinweg nicht erzielbar. Bei Laserstrahlung mit gauß'scher Energieverteilung ist es notwendig, während der Behandlung die einzelnen Spots zu überlappen, um ein angenähert kontinuierliches Behandlungsergebnis über die gesamte zu behandelnde Fläche hinweg zu erzielen. Das allerdings ist sehr schwer zu bewerkstelligen, hängt weitestgehend vom Feingefühl des Operateurs ab und kann insbesondere bei einer unkontrollierten Überlappung der Randgebiete zu einer Summierung der in die Hautpartien eingetragenen Energien an einzelnen Stellen des Behandlungsareals führen, durch die die Haut stärker als erwünscht geschädigt werden kann. Außerdem dauert die Behandlung um so länger, je weiter die einzelnen Spots sich überschneiden müssen.

In der Offenlegungsschrift DE 44 29 193 A1 ist eine Vorrichtung zur Erzeugung einer querschnittshomogenisierten Laserstrahlung angegeben, die als medizinisches Handstück im Sinne der vorliegenden neuen Erfindung ausgebildet ist. Mit dieser Vorrichtung kann eine modenhomogenisierte und räumlich homogenisierte Strahlung erzeugt werden, wie sie beispielsweise zur Abtragung der Hornhaut des Auges benötigt wird.

Als Strahlungsquelle wird hierbei ein gepulster Festkörperlaser mit einer Emission im Wellenlängenbereich 2µm bis 3µm eingesetzt. Die Pulsenergie liegt zwischen 100 µJ und 1J. Zur Übertragung der Energie von der Laseranordnung zum Handstück ist eine Faser vorgesehen, die eine Länge von mindestens 0,2 m und einen Durchmesser zwischen 50 und 1000 µm aufweist. Innerhalb des Handstükkes ist, der Faser nachgeordnet, ein transparenter, im Querschnitt kreisrunder Stab aus Quarz, Saphir oder YAG vorgesehen.

Durch die Kombination der Faser mit dem nachgeordneten transparenten Stab wird an der Abstrahlfläche eine Strahlung mit einem rotationssymmetrischen intensitätsprofil erzielt, bei dem die vom Laser abgestrahlten Modengemische effektiv in das homogenisierte radialsymmetrische Strahlprofil, beispielsweise mit gauß'scher, parabolischer oder ringförmiger Intensitätsverteilung, umgesetzt werden.

Damit ist dieses Handstück jedoch für Anwendungen ungeeignet, die, wie oben beschrieben, eine gleichmäßige Energieverteilung über den gesamten Strahlungsquerschnitt erfordern.

*In WO 95*/*18984 ist eine Einrichtung beschrieben, die mit einer Laserstrahlungsquelle verbunden ist, wobei zur Konvertierung der Intensitätsverteilung ein Prisma in den Strahlengang gestellt ist, das zwei Facetten aufweist, die gegeneinander geneigt sind und die bewirken, daß die Laserstrahlung in zwei Hälften mit jeweils unterschiedlichen Richtungen abgelenkt werden. Beide Hälften der Laserstrahlung werden nachfolgend ineinandergeführt, so daß sich innerhalb der vereinigten Laserstrahlung eine Intensitätsverteilung ergibt, die einer Überlappung zweier Gaußverteilungen entspricht.*

*Die Offenlegungsschrift* DE 196 32 749 A1 *offenbart eine "Optik zur Profilierung von Laserstrahlen", die sich auf Mikrolinsen bezieht, deren geometrische Anordnung und deren Durchmesser einem Gesetz zur Erzielung einer Gaußverteilung folgen. Dieser Veröffentlichung ist zu entnehmen, daß eine Laserstrahlung, die von einem Excimerlaser ausgeht, nach Durchgang durch eine solche Optik eine Gauß-Verteilung haben soll. Eine Homogenisierung der Strahlungsintensität über den gesamten Querschnitt ist demzufolge mit dieser Optik nicht zu erreichen.*

### Beschreibung der Erfindung

Davon ausgehend liegt der Erfindung die Aufgabe zugrunde, ein Handstück der vorbeschriebenen Art dahingehend weiterzuentwickeln, daß an der Abstrahlfläche eine Laserstrahlung mit bis in die Randzonen des Strahlquerschnittes hinein gleichmäßiger Intensitätsverteilung verfügbar und die Geometrie des Strahlquerschnittes so gestaltet ist, daß die Gefahr der Beeinflussung des Zielareals durch ungewollten Energieeintrag wesentlich verringert wird.

Diese Aufgabe wird bei einem Handstück der vorgenannten Art dadurch gelöst, daß
- *innerhalb des Handstückes ein optisches Element vorgesehen ist, das eine im Mikrometerbereich strukturierte und dadurch mikrooptisch refraktiv wirksame Fläche aufweist,*
- *ausgebildet als ein Array aus sphärischen, asphärischen, zylindrischen und*/*oder elliptischen, hexagonal und*/*oder orthogonal angeordneten, konkav und*/*oder konvex geformten Mikrolinsen, wobei die Ausdehnung einer einzelnen Linse senkrecht zur Strahlungsrichtung 10*µ*m bis 1000*µ*m beträgt,*
- *so daß der Laserstrahl bei Durchgang durch die Mikrolinsen in eine entsprechende Vielzahl von Teilstrahlen aufgeteilt wird, und dadurch, da*ß
- *die Mikrolinsen so auf der Fläche ausgebildet und positioniert sind, da*ß *sie jeweils den Teilstrahlen eine bestimmte Richtung innerhalb des Laserstrahlquerschnitts geben und dadurch der Laserstrahl nach Durchgang durch die Mikrolinsen an dem Behandlungsareal eine veränderte Form seines Querschnitts und*
- *eine über seinen gesamten Querschnitt homogenisierte Strahlungsintensität aufweist.*

Bevorzugte Abmessungen für die *Mikrolinsen* sind Durchmesser von 0,35mm und Tiefen von 0,005mm. Das Verhältnis von Tiefe zu Durchmesser sollte den Wert 0,5 nicht überschreiten. Bei Linsenstrukturen sollte dieser Verhältniswert größer als 0,02 sein und bevorzugt im Bereich von 0,1 bis 0,3 liegen.

Passiert der Laserstrahl die so strukturierte Fläche, erfolgt durch die Vielzahl der *Mikrolinsen* eine Aufteilung der Strahlung in eine Vielzahl von Teilstrahlen, wobei die Anzahl der Teilstrahlen von der Anzahl der auf der Fläche vorhandenen Strukturelemente abhängig ist. Je feiner die mikrooptisch wirksame Struktur ausgebildet ist, um so gleichmäßiger und homogener ist die Strahlungsintensität über den gesamten Querschnitt der Laserstrahlung nach Durchgang durch die beschriebene Fläche verteilt. Mit anderen Worten: beim Passieren der mikrostrukturierten Fläche erfolgt die Transformation einer ungleichmäßigen Energieverteilung innerhalb des Strahlquerschnittes in eine bis in die Randbereiche des Strahlquerschnittes hinein vergleichmäßigte Energieverteilung.

Diese Vergleichmäßigung ist insbesondere bei Verwendung eines Rubinlasers als Strahlungsquelle notwendig und vorteilhaft, da dessen Strahlung bekanntermaßen eine stark inhomogene Intensitätsverteilung in ihrem Querschnitt aufweist. Dabei kommt noch hinzu, daß die Intensitätsvertellung in der Rubinlaserstrahlung nicht konstant ist, sondern sich von Spot zu Spot ändert, so daß es bei Verwendung des Rubinlasers ohne die erfingdungsgemäß vorgeschlagene Einrichtung leicht zu Verbrennungen kommen kann.

Mit der mikrostrukturierten Fläche wird nicht nur die beabsichtigte Vergleichmäßigung der Intensität innerhalb des Strahlquerschnittes erzielt, sondern je nach Ausbildung der einzelnen Strukturelemente kann weiterhin, sofern das beabsichtigt und gewünscht ist, auch die Richtung der einzelnen Teilstrahlen beeinflußt werden. Das heißt, ein aus einer Faser beispielhaft mit kreisrundem Querschnitt austretender Laserstrahl kann durch gezielt vorgegebene Ausgestaltung der einzelnen Strukturelemente in einen Laserstrahl mit quadratisch, rechteckig, sechseckig oder anderweitig geformtem Strahlquerschnitt übergeführt werden.

Werden nämlich quadratisch, rechteckig oder sechseckig geformte Strahlquerschnitte auf das zu behandelnde Hautareal gerichtet, lassen sich die einzelnen Spots ohne gegenseitige Überlappung und auch ohne unbehandelte Fehlstellen nebeneinander setzen. Mit dem Ausschluß von Überlappungen wird ein zu hoher, mit Ausschluß von unbehandelten Fehlstellen ein zu niedriger Energieeintrag vermieden und so das Behandlungsergebnis bedeutend verbessert.

Die Umformung des Strahlquerschnittes wird erreicht, indem die *Mikrolinsen* auf der mikrostrukturierten Fläche so ausgewählt, geformt und positioniert sind, daß den Teilstrahlen, vor allem den peripheren Teilstrahlen, eine Richtung innerhalb des Laserstrahlquerschnittes gegeben wird, die auf eine gewünschte Außenkontur des Querschnitts zielt. Die Teilstrahlen füllen also nicht mehr einen kreisrunden Strahlquerschnitt, sondern beispielsweise einen quadratisch geformten Querschnitt gleichmäßig aus (die Kreisabschnitte sind ausgespart).

Das erfindungsgemäße Handstück zeichnet sich also gegenüber dem Stand der Technik durch eine über den gesamten Querschnitt homogenisierte Intensität der Laserstrahlung an der Abstrahlfläche und außerdem durch eine angepaßte Querschnittsform der Strahlung aus.

Die mikrooptisch wirksamen Strukturen sind beispielsweise mit Hilfe von Elektronenstrahllithographie, Photolithographie oder lonenaustauschverfahren leicht herstellbar.

Hier werden gemäß der Fresnelschen Gleichungen (Zusammenhang zwischen Polarisation, Reflexion, Absorption) circa 96 % der Laserstrahlung eingekoppelt, wodurch der Energieverlust und damit auch die Wärmeentwicktung auf ein vertretbares Maß beschränkt ist.

In einer Ausgestaltung der Erfindung ist vorgesehen, daß der mikrooptisch strukturierten Fläche eine Einrichtung zur Strahlfokussierung vor- oder nachgeordnet ist. Mit dieser Einrichtung läßt sich die Größe des Strahlquerschnittes einstellen. Als derartige Einrichtung kann beispielhaft eine Sammellinse vorgesehen sein, die im Strahlengang vor oder nach der strukturierten Fläche positioniert ist.

Als Einrichtung zur Strahlfokussierung kann aber auch eine Zoomoptik vorgesehen sein, mit der es in einfacher Weise möglich ist, die Größe des Spots zu beeinflussen. Ist die Zoomoptik mit einer entsprechenden Stellautomatik gekoppelt, kann die Spotgröße unkompliziert während der Behandlung verändert werden.

In einer weiteren Ausgestaltung der Erfindung ist vorgesehen, daß das optische Element mit der mikrooptisch wirksamen Fläche als strahlführender Stab ausgebildet ist, in welchem die Strahlung durch Totalreflexion weitergeleitet wird. Der Stab verfügt über eine Einstrahlfläche und eine Abstrahlfläche für die Laserstrahlung; dabei ist die Einstrahlfläche mit der mikrooptisch wirksamen Struktur versehen. Der strahlführende Stab kann aus Quarzglas gefertigt sein. Die Größe und Querschnittsform können bei Einstrahlfläche und Abstrahlfläche voneinander abweichen. Vorteilhaft jedoch sollte die Einstrahlfläche kreisrund ausgeführt sein, dieser Querschnitt über wenigstens 90% der Länge des Stabes erhalten bleiben und erst auf dem verbleibenden Längenabschnitt eine Reduzierung und/oder Formänderung des Querschnitts vorgesehen sein.

Aufgrund der Totalreflexionen innerhalb des strahlführenden Stab wird eine weitere "Durchmischung" der nach dem Passieren der strukturierten Fläche vorhandenen Vielzahl der einzelnen Teilstrahlen erreicht und damit eine weitere Vergleichmäßigung der Strahlungsintensität, bezogen auf den Strahlquerschnitt, bewirkt.

Eine zusätzliche Beeinflussung der über den Querschnitt verteilten Strahlungsintensität läßt sich erzielen, wenn die strukturierte Fläche gewölbt, bevorzugt konkav, besonders bevorzugt aber auch konvex ausgebildet ist.

Die Abstrahlfläche kann sowohl einen kreisrunden als auch einen vieleckigen, wie beispielsweise quadratischen oder sechseckigen Querschnitt aufweisen.

***Es ist empfehlenswert,*** wenn zwischen der Abstrahlfläche und der zu behandelnden Hautfläche eine Schicht aus einem transparenten Gel, beispielsweise einem Ultraschallgel vorgesehen sein. Hiermit wird die Einstrahlung des Laserstrahls in die zu behandelnde Hautfläche durch Reduzierung der Reflexion und verminderte Streuung weiter optimiert. Dies führt auch dazu, daß für das Laserlicht geringere Energiedichten benötigt werden. Der Brechungsindex des Gels soll dem Brechungsindex der Haut angepaßt und das Gel sollte mindestens für die Wellenlänge des verwendeten Laserlichtes transparent sein.

Damit wird eine effektive Eintragung der Laserenergie in die Haut erzielt, da das von der Haut reflektierte Licht auf einen unwesentlichen Anteil reduziert wird, wodurch Nebenwirkungen, die sonst durch Verlustwärme entstehen, vermieden werden. Bevorzugt wird ein Ultraschallgel verwendet, das physiologisch unbedenklich und insofern für kosmetische Zwecke geeignet ist. Es besitzt außerdem gute Wärmeleitfähigkeit.

Durch das Gel wird die Gefahr der Beschädigung der Epidermis noch weiter verringert und die Bildung von Rauch und Geruch bei der Behandlung wird vermieden, da die zu behandelnde Hautstelle besser temperiert ist. Die Wirksamkeit des Gels kann noch weiter gesteigert werden, indem von der behandelnden Hautpartie vor Beginn der Behandlung eventuell vorhandener Haarwuchs entfernt wird.

### Kurze Beschreibung der Zeichnungen

In den zugehörigen Zeichnungen zeigen
- Fig.1: eine erste Prinzipdarstellung der erfindungsgemäßen Anordnung
- Fig.2: unterschiedliche Strahlquerschnitte
- Fig.3: eine zweite Prinzipdarstellung der erfindungsgemäßen Anordnung
- Fig.4: eine Ausführungsvariante des Stabes
- Fig.5: Querschnittsformen der Abstrahlfläche
- Fig.6: Gestaltungsvarianten der Einstrahlfläche

### Ausführliche Beschreibung der Zeichnungen

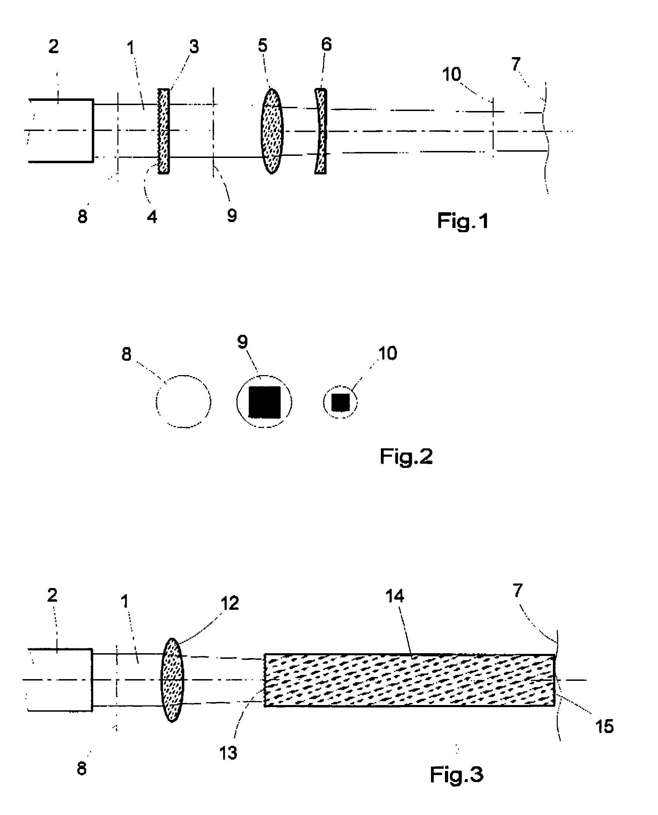

In Fig.1 ist die erfindungsgemäße Strahlführung und Beeinflussung der Laserstrahlung innerhalb des medizinischen Handstückes in einer ersten Ausgestaltungsvariante prinzipiell dargestellt. Im Strahlengang 1 der von einer Strahlführungseinrichtung 2, die sowohl als flexible Lichtleitfaser als auch in Form starrer Übertragungsglieder, die durch Gelenke miteinander verbunden sind, ausgestaltet sein kann, befinden sich ein optisches Element, beispielsweise eine aus Quarzglas bestehende Scheibe 3, die mit einer mikrooptisch wirksamen Fläche 4 versehen ist, und eine Zoomoptik, angedeutet durch die Linsen 5 und 6.

Die Laserstrahlung ist bei entsprechender Handhabung des Handstückes auf eine Hautpartie 7 gerichtet, etwa zum Zweck der Haarentfernung oder einer anderweitigen kosmetischen Behandlung.

Die Fläche 4 weist beispielhaft eine refraktiv wirksame Struktur auf, bei der eine Vielzahl konkav geformter sphärischer Linsen orthogonal zueinander angeordnet ist und die so in den Strahlengang 1 gestellt ist, daß der gesamte Strahlengang 1 diese Mikrolinsen passieren muß. Jede der Linsen hat dabei einen senkrecht zur Strahlungsrichtung gemessenen Durchmesser von etwa 0,35 mm und eine Tiefe von 0.005 mm.

Als Scheibe 3 kann beispielsweise ein von der Firma AMS Mikrooptik GmbH, Saarbrücken, Deutschland, angebotenes Mikrolinsenarray eingesetzt sein. Die Positionsabweichungen der einzelnen Linsen sind dabei kleiner als 0,2 µm. Beim Durchgang der Laserstrahlung durch die Mikrolinsenanordnung auf der Fläche 4 erfolgt die Aufteilung der Laserstrahlung in eine Vielzahl von Teilstrahlungen, die der Anzahl der Mikrolinsen entspricht.

Diese Aufteilung in die Vielzahl von Teilstrahlen bewirkt, daß der im Strahlengang 1 angedeutete kreisrunde Querschnitt 8 mit einer beispielhaft von einem Rubinlaser kommenden Strahlung ungleichmäßiger Intensitätsverteilung in eine Strahlung mit gleichmäßiger Intensitätsverteilung innerhalb eines quadratischen Querschnittes 9 transformiert wird (vgl. Fig.2).

Mit dieser quadratischen Querschnittsform wird nun die Strahlung auf die zu behandelnde Hautpartie 7 gerichtet, wobei mit Hilfe der Zoomoptik 5, 6 die Größe der auf die Hautpartie 7 auftreffenden Querschnittsfläche 10 beeinflußbar ist. Durch Variation der Zoomoptik beispielsweise läßt sich die Querschnittsfläche 10 vergrößern oder verkleinern. Damit ist eine Anpassung an die Fläche des zu behandelnden Areals unkompliziert möglich.

Ist das zu behandelnde Areal größer als die Querschnittsfläche 10, die mit der Zoomoptik 5,6 einstellbar ist, werden mehrere Spots auf dem Behandlungsareal 7 so nebeneinander gesetzt, daß das Behandlungsareal 7 nicht nur lückenlos abgedeckt ist, sondern auch eine Überschneidung der einzelnen Spots vermieden wird.

Fig.3 zeigt eine zweite Ausgestaltungsvariante der Erfindung, bei welcher der Strahlengang 1 der über die Strahlführungseinrichtung 2 eingekoppelten Laserstrahlung zunächst ebenfalls einen kreisrunden Querschnitt 8 mit inhomogener Verteilung der Strahlungsintensität aufweist. In diese Strahlung ist eine Sammellinse 12 gestellt, die den Laserstrahl auf die Einstrahlfläche 13 eines strahlführenden Stabes 14 fokussiert, der beispielhaft aus Quarzglas mit einer Länge von 55 mm und einem kreisrunden Querschnitt von 8mm Durchmesser gefertigt sein kann.

Die Einstrahlfläche 13 ist, wie bereits oben anhand der Fläche 4 beschrieben, mit einer Struktur aus nebeneinander angeordneten Mikrolinsen versehen. Dabei wird die Laserstrahlung auch hier beim Durchgang durch die Einstrahlfläche 13 in eine Vielzahl von Teilstrahlungen aufgeteilt und dadurch eine Homogenisierung der Intensitätsverteilung erzielt.

Innerhalb des strahlführenden Stabes 14 wird die Laserstrahlung durch Totalreflexion weitergeleitet, wobei eine weitere Homogenisierung erzielt wird. Somit ist an der Abstrahlfläche 15, die auf das Behandlungsareal 7 aufgesetzt ist, ein Laserstrahl verfügbar, dessen Querschnitt eine bis in die Randbereiche hinein gleichmäßige Strahlungsintensität aufweist.

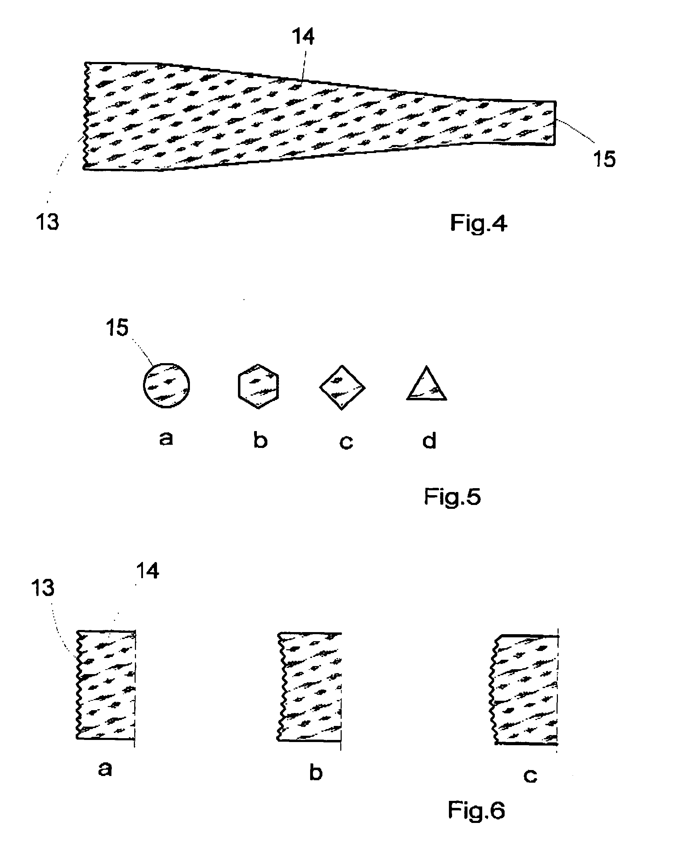

In Ausgestaltungen kann allerdings auch vorgesehen sein, daß sich der Querschnitt des strahlführenden Stabes 14 in Strahlungsrichtung kegelstumpfförmig verjüngt, wie das beispielhaft in Fig.4 dargestellt ist. Damit wird mit dem Stab 14 nicht nur eine verbesserte Homogenisierung erzielt, sondern zugleich auch eine Beeinflussung des Querschnitts der Laserstrahlung vorgenommen, indem die Abstrahlfläche 15 wie die Einstrahlfläche 13 einen kreisrunden Querschnitt aufweist, jedoch mit kleinerem Durchmesser (vgl. Fig.5a). In weiteren Ausgestaltungsvarianten ist es auch denkbar, daß die Abstrahlfläche 15 eine Querschnittsform aufweist, wie in Fig.5b bis 5d dargestellt ist, also einen sechseckigen, quadratischen oder auch dreieckigen Querschnitt.

In weiteren Ausgestaltungen der Erfindung ist die Einstrahlfläche 13, wie in Fig.6 dargestellt, plan (Fig.6a), konkav (Fig.6b) oder auch konvex (Fig.6c) geformt. Durch Zusammenwirken mit der strukturierten Einstrahlfläche 13 ist so eine weitere gezielte Beeinflussung der Intensitätsverteilung wie auch der Querschnittsform möglich.

## Patentansprüche

1. *Medizinisches Handstück, das mit einer Laserstrahlungsquelle verbindbar ist, von der ein Laserstrahl mit inhomogener Strahlungsintensität innerhalb seines Querschnitts ausgeht, und mit dem der Laserstrahl auf ein Behandlungsareal (7) gerichtet wird,* ***dadurch gekennzeichnet, da*ß**
- *innerhalb des Handstückes ein optisches Element vorgesehen ist, das eine im Mikrometerbereich strukturierte und **dadurch** mikrooptisch refraktiv wirksame Fläche (4,13) aufweist,*
- *ausgebildet als ein Array aus sphärischen, asphärischen, zylindrischen und*/*oder elliptischen, hexagonal und*/*oder orthogonal angeordneten, konkav und*/*oder konvex geformten Mikrolinsen, wobei die Ausdehnung einer einzelnen Linse senkrecht zur Strahlungsrichtung 10*µ*m bis 1000*µ*m beträgt,*
- *so da*ß *der Laserstrahl bei Durchgang durch die Mikrolinsen in eine entsprechende Vielzahl von Teilstrahlen aufgeteilt wird, und **dadurch**, da*ß
- *die Mikrolinsen so auf der Fläche (4,13) ausgebildet und positioniert sind, da*ß *sie jeweils den Teilstrahlen eine bestimmte Richtung innerhalb des Laserstrahlquerschnitts geben und **dadurch** der Laserstrahl nach Durchgang durch die Mikrolinsen an dem Behandlungsareal (7) eine veränderte Form seines Querschnitts und*
- *eine über seinen gesamten Querschnitt homogenisierte Strahlungsintensität aufweist.*

2. *Medizinisches Handstück nach Anspruch 1,* ***dadurch gekennzeichnet, da*ß** *das optische Element als strahlführender Stab (14) ausgebildet ist, in welchem die Strahlung durch Totalreflexion weitergeleitet wird und der über eine Einstrahlfläche (13) und eine Abstrahlfläche (15) für die Laserstrahlung verfügt, wobei die Einstrahlfläche (13) refraktiv wirkend strukturiert ist.*

3. *Medizinisches Handstück nach Anspruch 2,* ***dadurch gekennzeichnet,*** *da*ß *die Abstrahlfläche (15) einen kreisrunden Querschnitt aufweist.*

4. *Medizinisches Handstück nach Anspruch 2,* ***dadurch gekennzeichnet, da*ß** *die Abstrahlfläche (15) einen vieleckigen Querschnitt aufweist.*

5. *Medizinisches Handstück nach einem der vorgenannten Ansprüche,* ***dadurch gekennzeichnet, da*ß** *die strukturierte Fläche (4,13) gewölbt ausgebildet ist.*

6. *Medizinisches Handstück nach einem der vorgenannten Ansprüche,* ***dadurch gekennzeichnet, da*ß** *dem optischen Element eine Einrichtung zur Strahlfokussierung, bevorzugt eine Sammellinse (12), vor- oder nachgeordnet ist.*

7. *Medizinisches Handstück nach einem der vorgenannten Ansprüche, **dadurch** gekenntzeichnet, da*ß *dem optischen Element eine Zoomoptik (5,6) nachgeordnet ist.*

## Claims

1. A medical handpiece, which is connectable to a source of laser radiation, from which a laser beam emanates which has an inhomogeneous radiation intensity within its cross-section, and by which the laser beam is directed to a treatment area (7), **characterized in that**
- an optical element is provided within the handpiece, said optical element comprising a surface (4, 13) which is structured in the micrometer range and thus has a microoptically refractive effect,
- said surface being provided as an array of spherical, aspherical, cylindrical and/or elliptical, hexagonally and/or orthogonally arranged microlenses having a concave and/or convex shape, the extent of any individual lens perpendicular to the radiation direction being 10 µm to 1000 µm,
- so that the laser beam passing through the microlenses is divided into a corresponding multiplicity of partial beams, and further **characterized in that**
- the microlenses are formed and positioned on the surface (4, 13) such that they respectively impart to the partial beams a specific direction within the laser cross-section and, thereby, the laser beam, having passed through the microlenses, has a modified cross-sectional shape at the treatment area (7) and
- has a homogenized radiation intensity over its entire cross-section.

2. Medical handpiece according to claim 1, **characterized in that** the optical element is provided as a beam-guiding rod (14) in which the radiation is transmitted by total reflection and which comprises an irradiation surface (13) and an emission surface (15) for the laser radiation, said irradiation surface (13) having a refractively effective structure.

3. Medical handpiece according to claim 2, **characterized in that** the output surface (15) has a circular cross-section.

4. Medical handpiece according to claim 2, **characterized in that** the output surface (15) has a polygonal cross-section.

5. Medical handpiece according to any one of the preceding claims, **characterized in that** the structured surface (4, 13) is arched.

6. Medical handpiece according to any one of the preceding claims, **characterized in that** a means for beam focusing, preferably a collecting lens (12), is arranged preceding or following the optical element.

7. Medical handpiece according to any one of the preceding claims, **characterized in that** zoom optics (5, 6) are arranged following the optical element.

## Revendications

1. Poignée à usage médical, reliée à une source de rayonnement de laquelle sort un faisceau laser à intensité de rayonnement non homogène dans sa section, au moyen de laquelle le faisceau laser est dirigé vers une surface à traiter, **caractérisée en ce qu'**
il est prévu à l'intérieur de la poignée un élément optique présentant une surface (4, 13) structurée dans le domaine du micron et présentant ainsi un effet réfractif dans le domaine du micron,
formée comme un réseau de micro lentilles sphériques, non sphériques, cylindriques et/ou elliptiques, disposées de façon hexagonale et/ou orthogonale entre elles, de forme concave et/ou convexe, l'extension d'une seule lentille perpendiculairement à la direction du rayonnement étant de 10 *m à 1.000 µm,
de sorte que le faisceau laser, en traversant les micro lentilles, soit divisé en un nombre de rayons correspondant et **en ce que**
les micro lentilles sont formées et positionnées sur la surface (4, 13) de telle façon qu'elles donnent à chaque rayon partiel une certaine direction dans la section du faisceau laser et que, de ce fait, le faisceau laser, après passage à travers les micro lentilles, présente sur la surface à traiter (7) une forme modifiée de sa section et
une intensité de rayonnement homogénéisée sur toute sa section.

2. Poignée à usage médical selon la revendication 1, **caractérisée en ce que** l'élément optique est constitué par un barreau (14) guidant le faisceau, dans lequel le rayonnement est transmis par réflexion totale et qui dispose d'une surface d'entrée de rayonnement (13) et d'une surface de sortie de rayonnement (15) pour le rayonnement laser, l'entrée de rayonnement (13) étant constituée de façon à avoir un effet réfractif.

3. Poignée à usage médical selon la revendication 2, **caractérisée en ce que** la surface de sortie de rayonnement (15) présente une section circulaire.

4. Poignée à usage médical selon la revendication 2, **caractérisée en ce que** la surface de sortie de rayonnement (15) présente une section carrée.

5. Poignée à usage médical selon l'une des revendications précédentes, **caractérisée en ce que** la surface structurée (4, 13) est bombée.

6. Poignée à usage médical selon l'une des revendications précédentes, **caractérisée en ce qu'**un dispositif de focalisation du faisceau, de préférence une lentille convergente (12), est disposé avant ou après l'élément optique.

7. Poignée à usage médical selon l'une des revendications précédentes, **caractérisée en ce qu'**une optique de zoom (5,6) est monté en aval de l'élément optique.
